# EUROPEAN PATENT SPECIFICATION

(11) **EP 1 362 509 B1**
(45) Date of publication and mention of the grant of the patent: **02.02.2005**
(21) Application number: 02388034.7
(22) Date of filing: 08.05.2002
(51) Int. Cl.: A01K 43/00, C12M 3/00

(54) **An embryonated egg harvesting apparatus**
Anlage zum Einsammeln von embryonierten Eiern
Dispositif de récolte d'oeufs embryonnés

(43) Date of publication of application: 19.11.2003
(73) Proprietor: SANOVO ENGINEERING A/S, DK-5210 Odense NV (DK)
(72) Inventor: Dolby, Henrik, 5500 Middelfart (DK)
(74) Representative: Indahl, Peter Jensen

(56) References cited:
- GB-A- 1 077 182
- US-A- 3 326 205
- US-B1- 6 286 455

## Description

The present invention relates to an embryonated egg harvesting apparatus comprising an egg holder and an inserting device for entering into the egg through a hole in the egg shell.

Such an apparatus is known from US patent 4,187,989. The egg holder is here a pair of articulated arms and an upper support plate, and the inserting device is shaped as a double-knife utilized to crack the egg shell from below, enter into the egg, and separate the two egg shell halves from one another to allow the contents of the egg to drop down through a chute and onto a vibrating screen separating the embryos from the liquid contents of the eggs.

Fertilized and embryonated bird's eggs are used as host in the production of some vaccines for humans, such as influenza vaccines, as well as many vaccines for animals, such as vaccines for Newcastle disease virus, rabies virus, poultry bronchitis, Egg Drop disease, Avian diseases, etc. Efficient vaccine production requires the growth of large quantities of virus, and chicken eggs are considered convenient hosts for this purpose.

Some viruses like influenza virus change sero-types and require production of new vaccines specifically adapted to confer immunity to the new sero-types. This requires sampling and quick growth on a large scale of the new virus strains.

To grow a virus the eggs are inoculated with a sample of the virus by injecting the virus into the allantoic cavity of each egg. The eggs are inoculated 8-12 days after the fertilisation and are left for incubation for another 2-3 days. Then they are refrigerated to stop embryo growth and opened for harvesting.

Depending on the kind of vaccine, either embryos or selected fluids are harvested. The rest of the egg is discarded.

The egg contains three different fluids, namely the yolk, that is encapsulated in the yolk sac in the centre of the egg, the amnion fluid, which is encapsulated in the amnion together with the embryo and the yolk sac, and the allantoic fluid situated closest to the shell. When harvesting the allantoic fluid and optionally also the amnion fluid it is considered important that the yolk sac is not punctured, as the fat in the yolk acts as an undesired emulsifier in the harvested fluid, and yolk in amounts of more than about 1% can render the fluid useless for vaccine production. However, in some cases of production of human vaccine a small amount of yolk can be tolerated as the harvested fluid has to be purified. The concentration of virus in the amnion fluid is lower than in the allantoic fluid. However, in the production of animal vaccine it is normally considered a requirement that both the allantoic and the amnion fluids have to be harvested.

Several apparatuses have been developed for performing harvesting of embryonated eggs. Examples hereof are described in US 3,698,458, US 4,187,989, and US 4,110,223. All of these known apparatuses, however, suffer from a number of disadvantages the most important being that the recovery rate is too low and that the risk of contamination is often severe. In fact the product losses by using these known apparatuses for harvesting inoculated eggs have been so significant, that use of the mechanical apparatuses have been unable to compete with manual harvesting of the eggs.

The manual process is however very labour intensive and it too involves fairly high rates of discard and waste. In the manual harvesting procedure the eggs are typically decapped by machine and placed tray by tray with the decapped end of the eggs facing upwards. The personnel handles each egg individually by lifting the egg and inclining the decapped end towards a receptacle. Then a pincher is manually stuck into the egg while aiming at the head of the embryo in order to reduce the risks of piercing the yolk sac. The fluids running out of the egg are collected in a small bowl and if the embryo drops out through the opening in the egg it is picked up and discarded together with the shell. In order to reduce the risk of contaminating the valuable fluids, the small bowl is used to collect the fluid from only 5 to 10 eggs, and when it has been manually checked that the collected contents seem free from contamination, the small bowl is emptied into a 1 litre bottle. When the bottle is full it is sent to viral analysis for verification of the purity of the collected fluids. About 5% of the bottles fail in this control and their contents are rejected. Part of the rejections is caused by contamination stemming from the outside of the eggs or from the personnel, and another part is caused by too high contents of yolk in the fluids.

It is a purpose of the present invention to provide an apparatus for harvesting contents from embryonated eggs with a high yield of harvested useful material.

In view of this the apparatus according to the invention is characterized in that the inserting device is a embryo lifting device which is movable into the egg to a predetermined end position in which a free end on the embryo lifting device is located inside the egg at a distance from an end of the egg shell.

When the embryo lifting device is moved into the egg it engages the embryo or a membrane and lifts it clear from the area of the rim of the hole inside in the shell. This clearance of the circumference of the hole prevents the embryo from blocking part of the allantoic fluid from the outlet, and the embryo lifting device thus allows all of the allantoic fluid to freely run out of the hole and be drained from the egg. The automatic movement of the embryo lifting device to the predetermined end position inside the egg results in accurate, repetitive movements with sufficient precise positioning of the embryo during the emptying of fluids from the egg. The risks of egg contents getting in contact with the outside of the egg shell and of egg shell remains tearing loose from the rim area of the shell and dropping down together with the fluid are minimized by the mechanized automatic lifting of the embryo. The resulting improved emptying of fluid from the egg and reduced contamination from the outside of the egg shell and from personnel leads to a considerably higher yield of useful material than is obtainable by the manual procedures or the previously used mechanical procedures. At the same time the very cumbersome manual work is dispensed with.

It is preferred that the distance between the free end of the lifting device and of the egg shell is adjustable as this allows for optimisation of the movement of the embryo lifting device so that service experience with the device can be utilized to improve the yield. The adjustability further makes it possible to adapt the movement in dependency of the size and/or age of the eggs. When the eggs are of larger size the embryo will normally be larger, and the size of the embryo also increases with the age of the egg. The larger size of the embryo can conveniently be adapted to by increasing the distance between the free end of the device and the closed inside end of the egg shell.

In a preferred embodiment the embryo lifting device is moved upwards through the hole in a lower end of the egg shell. By orienting the egg with the hole facing downwards and the lifting device moving upwards, the force of gravity is utilized to emptying the fluid from the egg. It is of cause possible to orient the hole in other directions and use supplementary means for removing the contents of the egg, but the use of gravity results in an advantageously simple apparatus with a minimum of parts that get in touch with the contents.

The distance from the free end of the embryo lifting device to an end of the egg shell is normally defined in relation to the closed upper end of the egg shell, but it is also possible to measure the distance from the lower end of the shell, and in this case it is preferred that said distance is at least 5 mm measured vertically upwards from the lower end of the egg shell at level with a rim of the hole. The distance of 5 mm allows clear passage for the allantoic fluid. A smaller distance is possible because the fluids are quite runny, but there is normally no need to restrict the distance to be less than 5 mm.

When said distance is measured vertically from an upper end of the egg shell it is preferably of at least the same size as the breadth of the embryo. The embryo lifting device can lift the embryo up inside the egg so that the embryo is pressed against the inside of the egg shell. In order to diminish the risk of breaking the yolk sac it is preferred that the end position at least allows the embryo to be transversely positioned on top of the embryo lifting device without being crushed against the inside of the shell.

It is technically possible to break the shell membrane at the same time as the egg is decapped by removing the shell in one end of the egg, but it is considered an advantage if the egg can be turned with the hole pointing in a downwards direction while the shell membrane is intact and keeps the contents in place inside the egg. With such an orientation of the egg, the shell membrane can easily be broken to allow gravitational escape of liquid by using a separate membrane opening member such as an air jet member, a knife member, a tearing member etc. However, for the sake of simplicity it is preferred that the embryo lifting device is shaped to pierce an egg membrane, such as the shell membrane or the amnion.

The shell membrane, and also the amnion in case amnion fluid is collected, has a tendency to stick to and enclose the free end of the embryo lifting device so that not all of the fluid to be drained out has access to the outlet hole and so that ambient air is barred from free flow to the interior of the membrane or into the amnion cavity and this can also restrict the outflow of fluid. In a preferred embodiment designed to avoid these problems the embryo lifting device is shaped with at least one drainage area extending downwards from the free end. The drainage area can typically be a local shape variation at the circumference of the lifting device, e.g. one or more channels in the periphery or one or more protrusions.

In one embodiment of the apparatus the embryo lifting device is controlled to move in direction of the end position in a first movement for piercing the shell membrane followed by a retraction of the device out of the hole, and followed by a subsequent second movement continued to the end position. The first movement can be short and be stopped when the shell membrane is opened and well before the free end of the lifting device reaches the area of amnion. A large portion of the allantoic fluid flows out during the retraction movement, and the curved back of the embryo will normally settle in the area of the opening in the shell membrane. During the subsequent second movement the free end of the lifting device contacts the amnion and embryo with a very low risk of contacting and breaking the yolk sac. When the embryo is lifted clear of the hole, the remaining portion of the allantoic fluid flows out.

Preferably, a cutting device on the harvesting apparatus cuts the hole in the egg shell prior to insertion of the embryo lifting device into the egg. By providing the harvesting apparatus with the cutting device, decapping is effected immediately before the actual emptying of the eggs without any extended intermediate resting time. This is a clear advantage over the prior art manual procedure where the decapped and thus very vulnerable eggs wait for extended time before the manual emptying, and during this time the contents within the shell are open for contamination.

The risks of contamination are further reduced in an embodiment according to the present invention where the egg is held with its end pointing mainly horizontally towards the cutting device during cutting of the hole in the shell, whereupon the apparatus turns the egg to a position with the hole pointing down towards the embryo lifting device. When the decapping occurs the egg is in a position where broken off shell remains cannot fall down onto the shell membrane but will fall clear of the egg, and in this manner any contamination present on the outside of the shell will not spread to the sterile contents inside of the membrane.

When the harvesting is for live virus in the embryo the apparatus can be embodied so that the embryo lifting device is tubular and has its end opposite the free end connected to a vacuum chamber for sucking the embryo into the vacuum chamber when the vacuum in the chamber is activated. Such extraction of the embryo through the lifting device ensures that the embryo completely avoids risks of contact with material that could have been contaminated by the outside of the egg shell.

In an embodiment the apparatus has a screening station for detecting eggs without an embryo and preferably also a discard device preventing the embryo lifting device from entering such eggs. By screening for eggs without an embryo it is possible to prevent the lifting device from moving directly up into the yolk sac and cause extended yolk contamination of the collected liquids.

By the following examples of embodiments the invention will be explained in more detail with reference to the accompanying drawings, of which
Fig. 1 depicts a cross section of an embryonated egg,
Fig. 2 illustrates processing steps performed by an embodiment of an egg harvesting apparatus according to the present invention,
Fig. 3, 3a - 5, 5a outline three different lifting devices for harvesting the fluids only, seen in side view and in cross section, respectively,
Fig. 6, 6a show another embodiment of a lifting device seen in side view and from above, respectively, for harvesting the fluids only,
Fig. 7 shows a cross section of a lifting device for harvesting the embryo, and
Fig. 8a - 13b outline embodiments of the apparatus according to the invention.

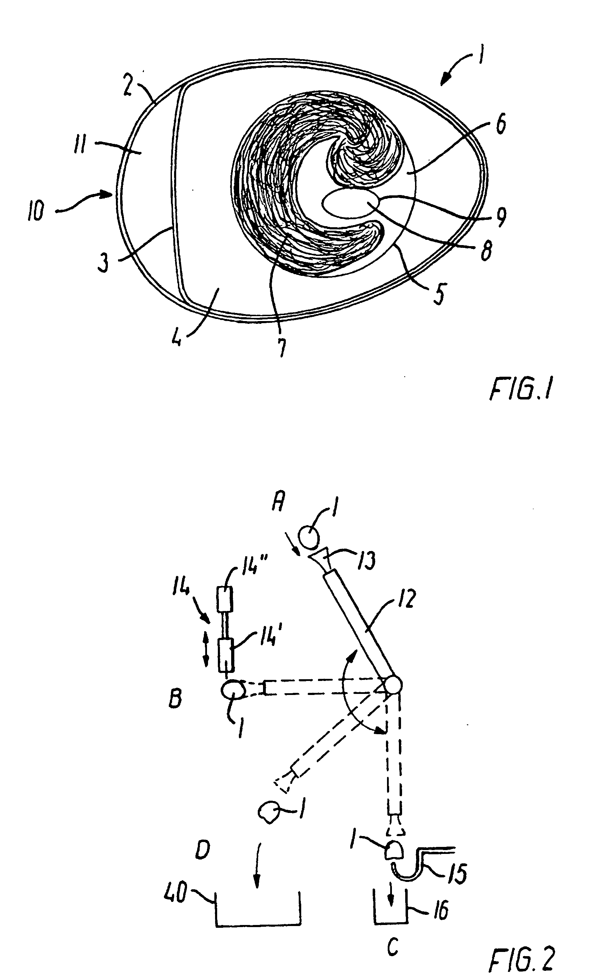

In Fig. 1 a fertilized egg 1 is shown in cross section. Just inside an egg shell 2 and a shell membrane 3 is the allantoic fluid 4 surrounding the amnion 5. The amnion contains the amnion fluid 6, the embryo 7 and the yolk 8, which is contained in the yolk sac 9. The embryo 7 is curved around one side of the yolk 8 and normally turns its back upwards, provided that the egg has not recently been turned. In the wide end 10 of the egg an air pocket 11 is present between shell 2 and shell membrane 3. The size of this air pocket 11 grows with the age of the egg.

Eggs used in the production of vaccine for animals are preferably produced under strictly controlled conditions and normally the so-called SPF eggs laid by Specific Pathogen Free chickens are used.

Fig. 2 shows a harvesting apparatus comprising a holding arm 12 that receives the egg 1 in a position A. The holding arm 12 has an egg holder 13 which can be a suction head of conventional type that by vacuum action can grip and hold the egg 1, normally at the narrow end of the egg. Other types of egg holders for controlled gripping and holding of eggs can be used, such as mechanical gripping means or adhering means, and the egg holder can optionally be supplemented with egg support means for stabilizing the egg position during decapping. The egg support means can e.g. be plate walls, or several elongate rods 50 mounted on a base plate 61 which is mounted onto a suction pipe 62, e.g. by welding or another kind of fixing. The suction pipe continues up through rod 12 to a vacuum source provided with a control valve for applying or removing vacuum to the suction head 13, which has a bellow-shaped portion and is made of a flexible material, such as a resin or rubber.

From the loading position A, the holding arm 12 is swung to a second position B, where the top of the egg shell is chopped off by means of a cutting device 14. The cutting device 14 can include a sawing means 14' moved by an actuator 14'', or a mechanical chopper device which can include a movable knive, or a more sophisticated means such as a laser cutter, e.g. of the type disclosed in US patent 5,285,750. Here and in the following the top of the egg 1 is to be understood as the wide end, where the air pocket 11 is located. The decapping or chopping is preferably done in such a manner, that the shell membrane 3 is left un-broken. The holding arm 12 has in its second position B preferably been swung downwards at least to a horizontal position or further downwards with the top of the egg pointing at least slightly downwards, thereby ensuring that possible loose shell fragments fall away from the egg and not onto the membrane.

The egg is now swung to a third position C where the holding arm 12 is essentially vertical and the bare membrane at the chopped-off end faces downwards. The turning of the egg occurs relatively fast not giving the embryo 7 sufficient time to turn inside the egg 1. The desired content of the egg is harvested in position C and the remaining parts of the egg is then swung to a fourth position D of the holding arm and discarded. There are two reasons that the discard position D is arranged between the positions B and C; one is that the discarded parts may all be dropped in one area possibly using the same waste receptacle 40, the other that the monitoring of the harvesting, as will be explained in more detail later, is made easier when the harvesting position is an outermost position in the apparatus.

The apparatus in Fig. 2 processes only one egg at a time. However, normally it is a desire to process several or many eggs in simultaneous operations to achieve an economical production. One way of achieving this is by arranging a row of egg holders etc. as those illustrated in Fig. 2 next to each other, but it may also be achieved with egg holder units etc. holding a plurality of rows of eggs at a time as will be explained later.

For harvesting the contents of the egg an embryo lifting device 15 can be moved up into the egg 1 from below. The lifting device 15 breaks the shell membrane 3 and lifts the embryo 7 and the yolk sac 9 up towards the inside of the narrow end of the egg. As will be explained later the lifting device may lift the embryo and yolk a bit before it breaks the shell membrane utilizing the elasticity of the membrane. Once the shell membrane is broken, the allantoic fluid and possibly the amnion fluid will start to drain from the egg into a collecting means 16 located underneath the egg. If the shell membrane were simply broken and there were no subsequent lifting of the embryo and yolk, the embryo would fall down and block or obstruct the hole in the shell membrane thereby making it difficult for the remaining fluid to escape from the egg.

When the lifting device 15 is moved up into the egg 1 the shell membrane 3 may stick to the device surface so that the edge of the shell membrane is bent upwards forming a circular trough around the lifting device. If the lifting device is only moved a small distance up into the egg the shell membrane may completely cover the part of the lifting device that is inside the egg leaving no room for the fluids to drain. This can be prevented by designing the lifting device with movable cutting means cutting up the membrane, but it makes the lifting device more complicated. Therefore, preferably the lifting device 15 lifts the embryo 7 and yolk sac 9 a distance of at least 5 mm. Too large a distance may, however, also entail disadvantages as the embryo and yolk sac could potentially be crushed against the inside of the egg shell. To avoid this, the distance is preferably not larger than 30 mm. The distances given herein are suitable when working with chicken eggs, however, if eggs from a much larger or a much smaller bird are used the minimum and maximum distances may be altered proportionally.

When the lifting device 15 is retracted from the egg 1 any shell membrane 3 sticking thereto and forming the circular trough mentioned above will be drawn downwards enabling a draining of the contents of the trough. The volume of this trough is normally sufficiently small for it to be drained completely during the retraction of the lifting device.

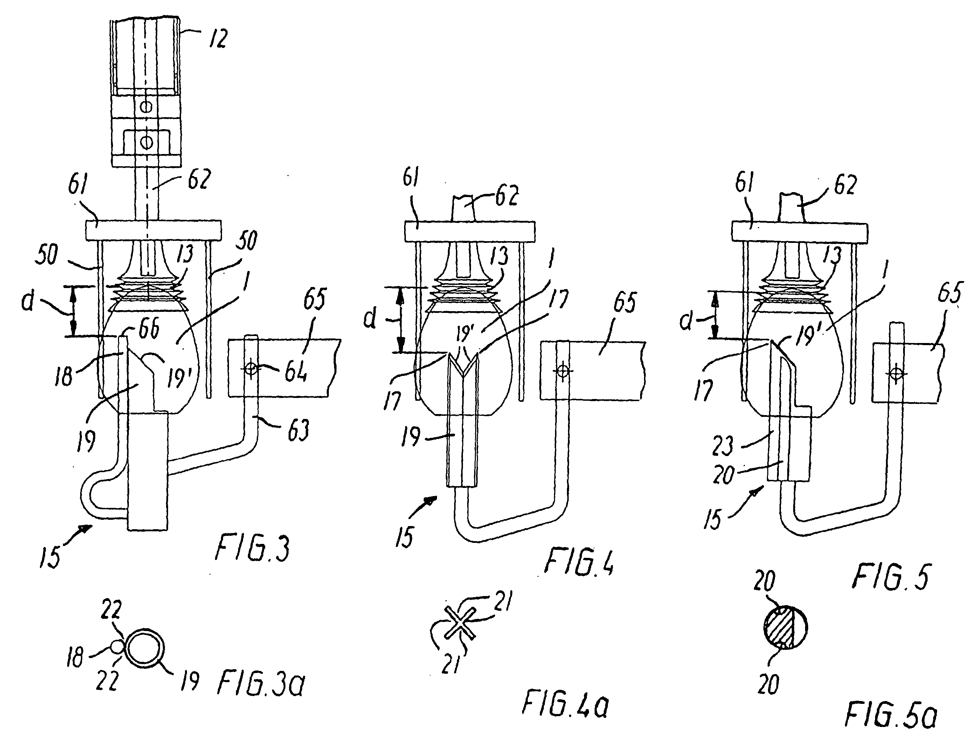

For some vaccines, typically for animal use, the amnion fluid is to be harvested and the lifting device 15 is then designed also to break the amnion 5. The embodiments of Figs. 3 and 4 are designed for this.

In the following description of different embodiments the same reference numerals are for the sake of simplicity used to describe alike features.

Figs. 3, 4, and 5 show three different embodiments of lifting devices 15 seen from the side. The lifting devices 15 of Figs. 4 and 5 have a free end shaped as pointed tips 17 for penetrating shell membrane 3. In both cases each tip 17 of the lifting device is transversely displaced relative to the centre of the egg 1 to minimise the risk of causing damage to the embryo or the yolk sac. The lifting device of Fig. 3 has an elongate part 19 which at its upper end has a cutting edge 19' for penetrating and opening up shell membrane 3. Part 19 is mounted on a carrier 65 by a fixture 63 and a fixing organ 64, such as a screw or a bolt. Carrier 65 is driven by an actuated drive, such as a pneumatic cylinder unit, or an eccentric cam with a motor drive. The drive can move the carrier in a linear movement between a position in which the lifting device 18, 19 is located fully outside the egg, and another position in which it is in a predetermined end position inside the egg. In this end position a free end 66 or 17 on the lifting device 19 is located at a predetermined distance d from the small end of the egg shell. The distance d is adjustable.

The cross sectional shape of the lifting devices 15 is shown in Figs. 3a, 4a, and 5a. In the shown embodiments the cross sections are all non-circular thereby ensuring, that the shell membrane and possibly the amnion cannot close sealingly about the lifting device as would be the case had it been of a purely circular cross section. In the embodiment of Fig. 5a recesses 20 in the elongate part 19 extend downwards from just below cutting edge 19' and constitute drainage areas, in the embodiment of Fig. 4a the elongate part 19 has the cross sectional shape of a cross, and in the embodiment of Fig. 3a a round rod 18 of a relatively small diameter and with the flat free end 66 is attached to the outside of part 19, the cross section thereby constituting a figure of eight. Thus, the fluids can leave the egg via one of the drainage areas formed by the channels that are present between the shell membrane and one or more parts of the lifting device, i.e. via the recesses 20 in Fig. 5a, via the drainage areas at corners 21 at the centre of the cross in Fig. 4a, or via V-shaped drainage areas 22 at the transition between the two parts 18, 19 in Fig. 3a. Similarly air can be drawn into the egg via these drainage areas or channels. The elongate part 19 is tubular. The elongate part 23 of Figs. 5, 5a can also be formed as tubes, and the tubes can have depressed or stamped areas constituting the drainage areas.

The upper end of part 19 is bevelled so that the cutting edge 19' is sloped and forms an angle with a horizontal line. The angle is normally in the range from 20 to 85° and preferably from 40 to 50°. The upper end of the cutting edge is pointed into tip 17. When the tip engages the shell membrane during the movement into the egg it will firstly cause a certain deflection of the membrane, but due to the tip being pointed, the tip contact area against the membrane will be suitable small to result in a sufficient large force to cut through the membrane at a moment where the membrane deflection is suitably small. This quick penetration is advantageous because it leads to a gentle opening for outflow of liquid from the egg. If the membrane is allowed to heavily deflect before penetration, it will abruptly disrupt followed by uncontrolled vigorous outflow of liquid with the risk that liquid drops contact the outside of the shell and then drops down as harvested, polluted fluid. When the membrane is penetrated before considerable membrane forces are built up by the deformation, the opening of the membrane does not release unduly great forces. As an alternative to an oblique cutting edge with a pointed tip, the upper end of part 19 could have a needle-like projection for penetrating the membrane and a practically horizontal cutting edge. It is also possible to open the membrane by a member that is movable in relation to part 19, such as a needle-like projection moved along a circular path corresponding to the desired diameter of the hole in the membrane.

The upper end of part 19 is preferably shaped with a cutting edge that does not cut loose a portion of the membrane, but only breaks the membrane open and cuts it into one or more flaps that maintain the connection to the remaining membrane.

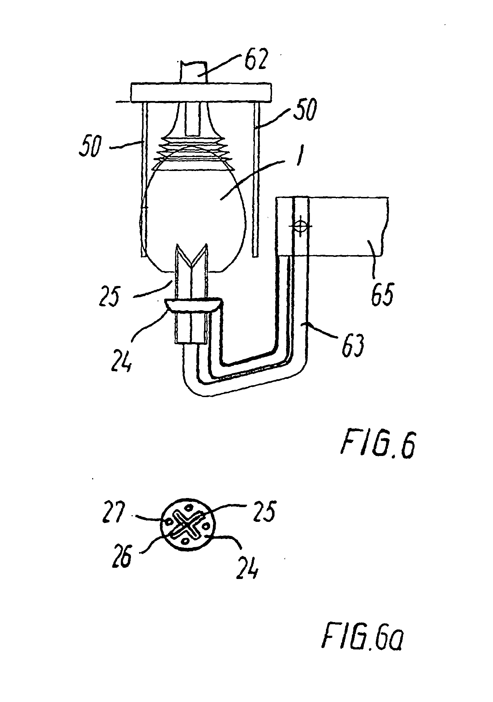

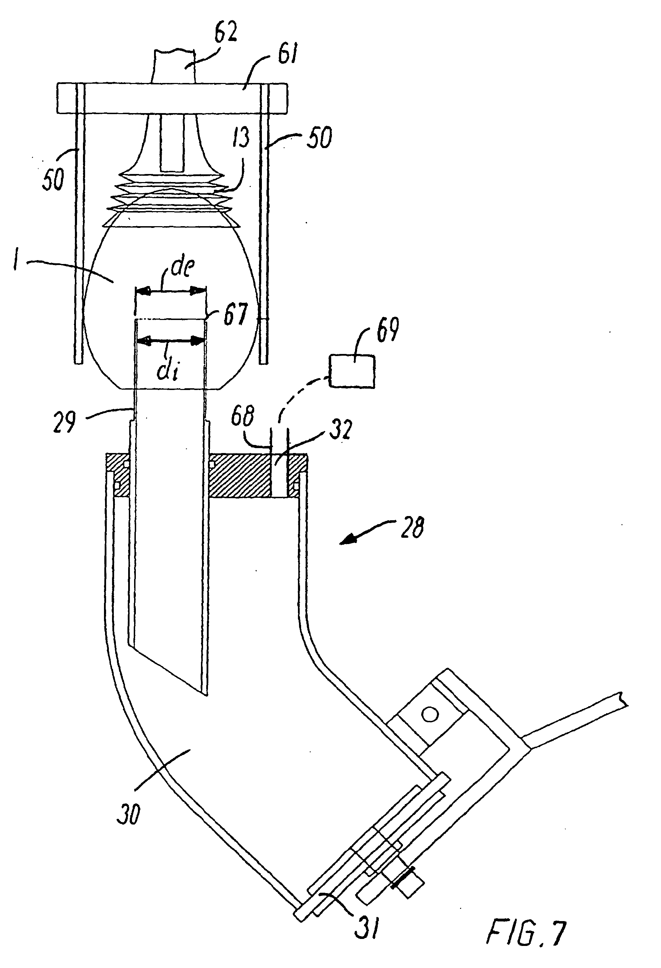

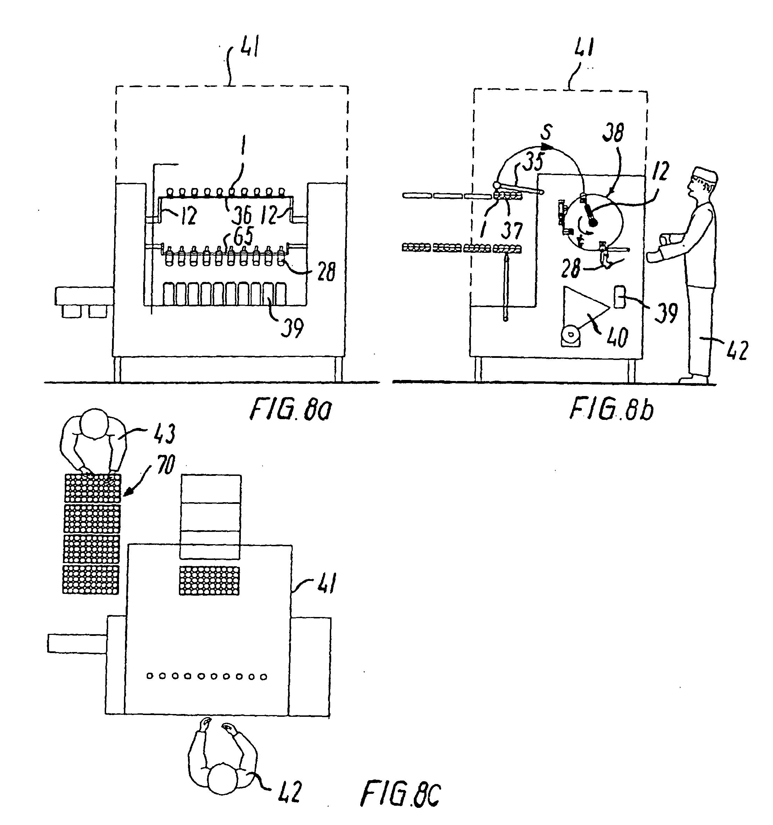

Fig. 6 shows another embodiment of a lifting device 24, which is shaped as a spoon and is associated with a separate penetration device 25 provided for penetrating the shell membrane 3. The harvesting of the egg 1 using these devices 24, 25 is a two-step process involving the penetration of the shell membrane by the penetration device 25 and a subsequent lifting of the embryo by the lifting device 24. After the penetration of the shell membrane the allantoic fluid is allowed to drain from egg until the embryo settles on the shell membrane blocking the hole or obstructing outflow of part of the fluid. Then the embryo is lifted by the lifting device 24 to allow the rest of the fluid to escape. This two phase draining allows the use of a lifting device 24 with no sharp or pointed part that might potentially cause damage to the embryo or the yolk sac. Thus this lifting device 24 may be used without risks of contamination by release of yolk irrespective of how the embryo and yolk sac are positioned within the egg relative to the opening.

The composed lifting device 24, 25 is seen from above in Fig. 6a. In the embodiment illustrated, the penetration device 25 is shaped like a cross resembling the lifting device in Fig. 4 and 4a, and it is mounted at the centre of the spoon-shaped lifting device 24 working trough opening 26 shaped like a cross. The lifting device 24 has holes 27 for draining the fluids from the embryo. However, the cross-shaped opening 26 also serves as draining so that the lifting may be made without the extra holes 27.

The harvesting in a two-stage process may also be performed with a lifting device as described in Figs. 3 to 5 performing both the penetration and the lifting step. This way of using the lifting devices 15 is advantageous over the one-phase draining in that the engagement between the embryo and the lifting device will be more well defined because after opening of the membrane the embryo is floating down to rest on the shell membrane instead of floating in a fluid, and the positioning of the embryo will thus be more well defined, which will improve the possibilities of contacting the embryo in a manner involving only a very low risk of breaking the yolk sac. The two-stage process may also be performed by using a penetration device that is completely separate from the lifting device, such as a nozzle ejecting an air jet cutting through the membrane, or a mechanical means such as a knife.

During operation the lifting device is moved into the egg to a predetermined end position, which can typically be located in the range of from 10 to 35 mm, and suitably from 25 to 30 mm, from the rim of the hole in the egg shell. Other distances are of course also possible. The predetermined end position can be adjusted in accordance with the size of the egg, the age of the egg, experience with emptying the harvestable fluids from the egg, etc. The free end of the lifting device is then located at a distance from the closed end of the egg of at least 5 mm.

If the embryo 7 is to be harvested the lifting device can be designed to suck the embryo out, as will be explained in greater detail below, or by some other means for removing the embryo from the egg 1, such as by shaking the egg, by mechanically scraping out the embryo or any other method which is non-damaging to the embryo. Depending on the method used, some of the fluids will be emptied from the egg together with the embryo. These fluids can subsequently be separated from the embryo.

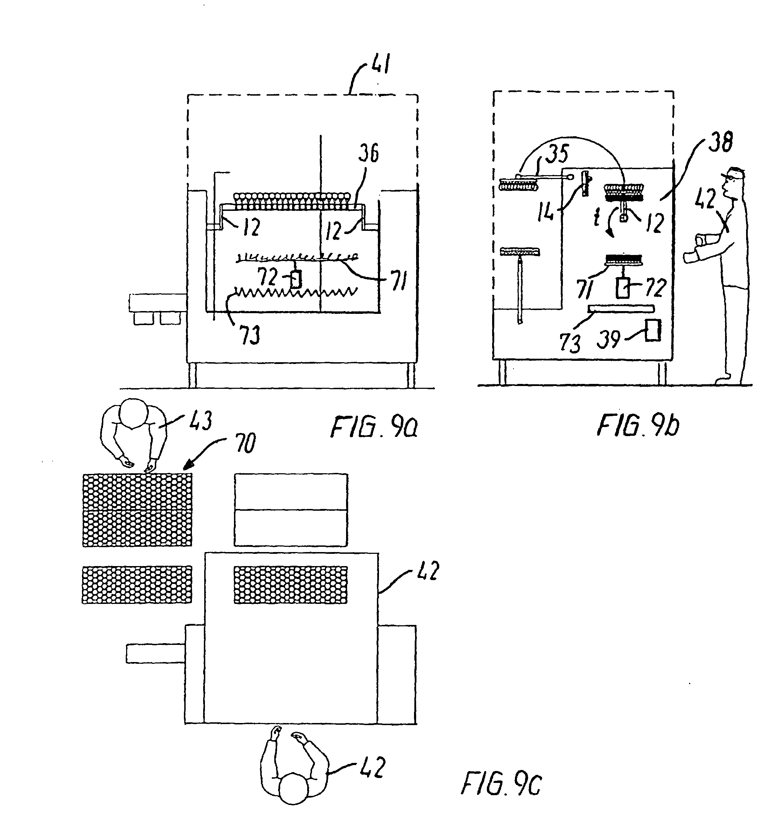

Fig. 7 shows a cross section of a lifting device 28 for harvesting the embryo from egg 1. The lifting device 28 includes a tubular member 29 that is connected to a chamber 30, which has an end closure 31 at its bottom. When harvesting the embryo the tubular member 29 is moved up into the egg. There can be used two different procedures for breaking the shell membrane. In a first procedure the tubular member is moved sufficiently far into the egg to penetrating the shell membrane by mechanical action. In a second procedure the lifting device 28 is moved upwards until a free end 67 formed by the annular rim of tubular member 29 is in contact with the shell membrane and has lifted the membrane somewhat upwards. The membrane is lifted sufficiently far to be in full contact with the rim but not so far as to break the membrane. Then a vacuum is applied to the chamber 30, and this vacuum causes breaking of the membrane.

After breaking the membrane a vacuum applied to chamber 30 sucks the embryo through the tubular member 29 and into the chamber 30. The chamber 30 preferably has a curved shape to gently brake the fall of the embryo. The curved shape of the tubular member also assists in avoiding that liquid within the member is sucked up through an opening 32 connected with a pipe 68 which is connected to a source of vacuum 69. The vacuum is activated by a valve that e.g. can be controlled by a sensor activated by the lifting device assuming the position with its free end moved into the egg. The vacuum is preferably not too forceful. A vacuum in the interval of 0.1 to 0.2 bar below atmospheric pressure (an absolute pressure of about 0.8 to 0.9 bar) is preferred because it does not cause damage to the embryo. When the pressure inside the chamber 30 is sufficiently normalised the end closure 31 is opened to allow the embryo to drop out of the chamber 30 onto a collection unit (not shown).

It is preferred that the tubular member 29 has an inner diameter dᵢ in the range from 15-20 mm, preferably in the range from 17-19 mm. If the inner diameter is smaller than 15 mm it is more difficult to suck the embryo through tubular member 29 without damage to the embryo, and if the diameter is larger than 20 mm, the external diameter dₑ of tubular member 29 becomes so large that the decap hole in the egg shell must be so large that it can become required to break the membrane during decapping. Although this is possible, it is preferred that the membrane is kept intact until the actual harvesting is taking place. With an inner diameter in the range from 17-19 mm the decap hole can have a diameter in the range of 22-23 mm. The tubular member is preferable recessed in its upper portion in order to provide the maximum clearance between the outside of the tubular member and the shell rim of the hole.

All of the lifting devices described above are shown as working vertically, but they may also be employed in an inclined manner where the eggs are held e.g. at an angle different from the vertical, such as an angle of about 45°.

Fig. 8 shows a general outline of an apparatus for harvesting e.g. 10 eggs at a time, however, this number may conveniently be altered to correspond to the number of eggs in a row of a particular type of egg trays. As may be seen from Fig. 8a the apparatus has one yoke 36 carrying a row of egg holders with eggs 1 and being mounted on arms 12, and another yoke 65 carrying a row of lifting device the lifting device each being positioned directly below one of the eggs. Fig. 8b shows the apparatus seen from the side the arrow s showing how a egg carrying yoke 35 swings the eggs from a tray 37 to a loading position corresponding to the position A of Fig. 2. The harvesting unit 38 corresponds to the unit in Fig. 7. Receptacles 39 and 40 are arranged beneath the harvesting unit for receiving the harvest and waste, respectively. Preferably the entire apparatus is contained within a housing 41 where a flow of sterile air within the housing ensures that the harvest is not contaminated with bacteria from the surroundings.

The harvesting process can be monitored by a person 42 standing outside the house looking through a window as illustrated in Figs. 8b and 8c. As the harvesting position is the uttermost position of the harvesting unit, as explained in the description of Fig. 2, the person monitoring the harvesting has the best possible overview over the row of receiving receptacles 39 (illustrated by the row of eggs in Fig. 8c) .

In principle the one person monitoring the harvest is sufficient to obtain a well functioning harvesting process. However, it is often preferable, that a second person 43 supervises the loading of the egg filled trays 37 into the apparatus. The person 43 is preferably standing at a screening station 70 where the eggs are illuminated so that the embryo is clearly discerned as a shadow within the egg. Eggs without shadow are removed before loading onto the egg holders.

In a preferred embodiment the trays 37 are fed to apparatus from a position in which the supervisor may inspect them standing on the floor and then lifted to a higher position where the eggs are lifted up from the tray one row at a time as illustrated in Fig. 8b and described above. The various devices, sensors and drives in the apparatus are mounted in a normal machine frame standing on the floor and is encapsulated by the housing.

Fig. 9 shows an alternative embodiment of the harvesting apparatus. In broad outline the apparatus works as the one in Fig. 8, only here the eggs are harvested one tray at a time instead of only one row. As best seen in Fig. 9c the trays depicted here are somewhat larger than the trays in Fig. 8, carrying 132 eggs instead of 50. Further the lifting device (Fig. 3-5) for harvesting the fluid are mounted on a grid-like frame 71 which by a drive 72 can be moved from the inactive position to the end position with the lifting devices partly inside the eggs. As an alternative to using one receptacle for collecting fluid from each lifting device it is also possible to use collecting devices can be a channel extending under each row of lifting devices. The channels can be formed by a bellows-bent plate 73 placed below the lifting device. The plate is slightly inclined and at the lower end there is placed a separate receptacle 39 for each channel.

The type of lifting device is chosen depending on the material to be harvested. The lifting devices etc. of one type can be exchanged with lifting devices etc. of another type according to the material to be harvested so that one and the same apparatus can be adapted according to the actual type of harvesting. The holding and lifting devices can also be changed for others designed for a different number of eggs at a time.

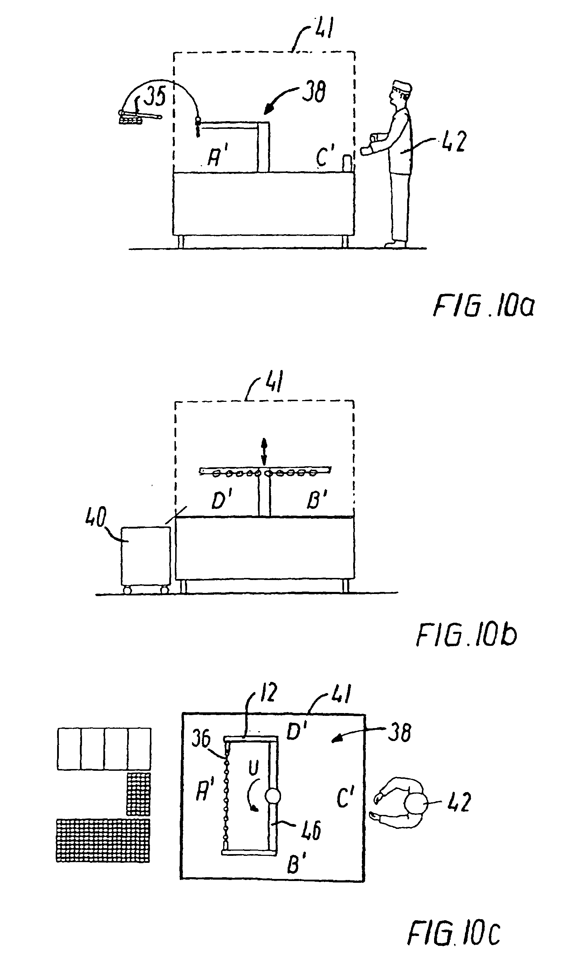

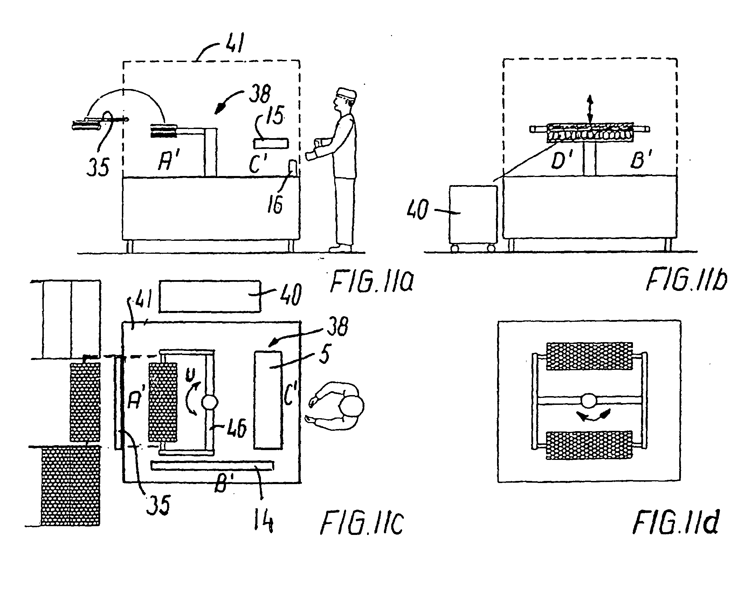

Two further embodiments of the harvesting apparatus are shown in Fig. 10 and 11. These apparatuses differ from the former two by the swinging motion of the harvesting unit. In Figs. 8 and 9 the holding arms 12 swing in a vertical plane as indicated by the arrows t while the holding arms 46 in Fig. 10 and 11 swing in a horizontal plane as indicated by the arrow u. The positions corresponding to the positions A, B, C and D in Fig. 2 is indicated by A', B', C' and D' in Fig. 10 and 11. As in Figs. 8 and 9, Figs. 10 and 11 differ by the type of trays being used and the fact that the apparatus in Fig. 10 handles on row at a time while the apparatus in Fig. 11 handles an entire tray.

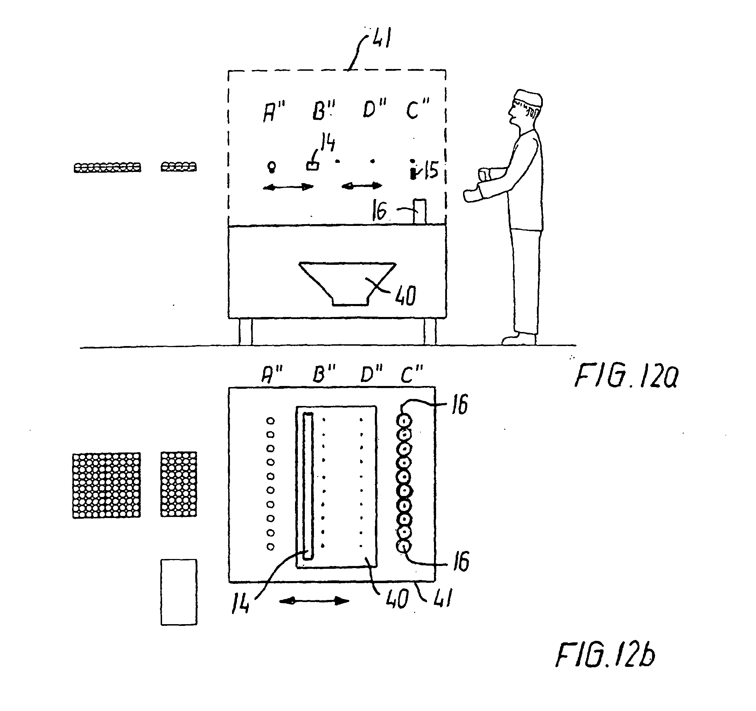

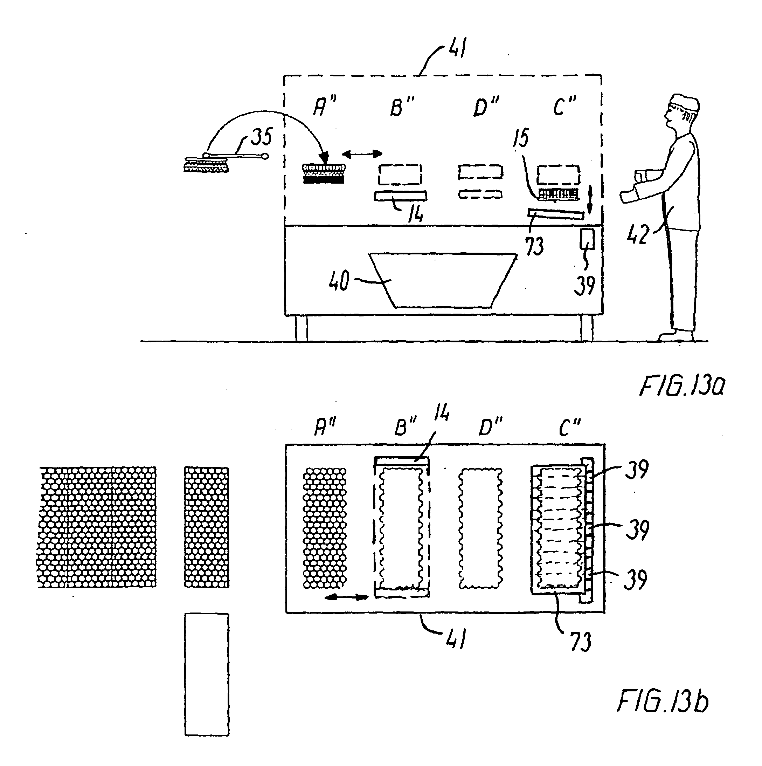

Two still further embodiments of the harvesting apparatus are shown in Fig. 12 and 13. These apparatuses differ from the former four by the fact that the harvesting unit works by a linear motion instead of a swinging one. The eggs 1 are picked up and swung to the position A" by means (not shown) corresponding to ones in Figs. 8 to 11. They are then copped in the position B", harvested in the position C" and the remains are discarded in position D". As for Figs. 8 and 9 and Figs. 10 and 11, Figs. 12 and 13 differ by the type of trays being used and the fact that the apparatus in Fig. 12 handles on row at a time while the apparatus in Fig. 13 handles an entire tray. The linear motion can be effected by mounting the various devices on chains of the like that pull the devices back and forth while their position is guided, e.g. by guide taps engaging guide tracks.

In Figs. 8 to 13 the apparatuses are shown holding eggs only in the receiving position indicated by A, A' and A''. The apparatuses shown in Figs. 10 and 11 may however be equipped with suitable extra holding means as shown in Fig. 11d making the carousel symmetrical holding eggs in the positions C, C' and C'' at the same time thereby enabling an increased output.

Details from the various embodiments can be combined into other embodiments. Although cutting at the wide end is preferred, the hole cut in the egg need not be in the end of the egg, but can be made anywhere in the egg shell.

## Claims

1. An embryonated egg harvesting apparatus comprising an egg (1) holder (13) and an inserting device for entering into the egg through a hole in the egg shell, **characterized in that** the inserting device is a embryo lifting device (15) which is movable into the egg to a predetermined end position in which a free end on the embryo lifting device is located inside the egg (1) at a distance (d) from an end of the egg shell.

2. An embryonated egg harvesting apparatus according to claim 1 **characterized in that** said distanced (d) is adjustable, preferably adjustable in dependency of the size and/or age of the eggs (1).

3. An embryonated egg harvesting apparatus according to claim 1 or 2, **characterized in that** the embryo lifting device (15) is moved upwards through the hole in a lower end of the egg shell.

4. An embryonated egg harvesting apparatus according to claim 3, **characterized in that** said distance (d) is at least 5 mm measured vertically upwards from the lower end of the egg shell at level with a rim of the hole.

5. An embryonated egg harvesting apparatus according to claim **characterized in that** said distance (d) measured vertically from an upper end of the egg shell, is of at least the same size as the breadth of the embryo.

6. An embryonated egg harvesting apparatus according to any one of claims 1 to 5,**characterized in that** the embryo lifting device (15) is shaped to pierce an egg membrane (3).

7. An embryonated egg harvesting apparatus according to claim 6, **characterized in that** the free end of the embryo lifting device (15) is pointed.

8. An embryonated egg harvesting apparatus according to any one of claims 1 to 7, **characterized in that** the embryo lifting device (15) is shaped with at least one drainage area extending downwards from the free end.

9. An embryonated egg harvesting apparatus according to any one of claims 1 to 8, **characterized in that** the embryo lifting device (15) is controlled to move in direction of the end position in a first movement for piercing the shell membrane (3) followed by a retraction of the device out of the hole, and optionally followed by a subsequent second movement continued to the end position.

10. An embryonated egg harvesting apparatus according to any one of claims 1 to 9, **characterized in that** a penetrating device is controlled to move in direction of the end position for piercing the shell membrane (3) followed by a retraction of the device out of the hole, and that the lifting device (15) is moved to the end position.

11. An embryonated egg harvesting apparatus according to any one of claims 1 to 10, **characterized in that** the embryo lifting device (15) is inserted into the egg (1) for a predetermined holding time.

12. An embryonated egg harvesting apparatus according to any one of claims 1 to 11, **characterized in that** a cutting device (14) on the harvesting apparatus cuts the hole in the egg shell prior to insertion of the embryo lifting device (15) into the egg (1), which the cutting device (14) preferably decaps the shell but leaves the shell membrane (3) intact.

13. An embryonated egg harvesting apparatus according to claim 12, **characterized in that** the egg (1) is held with its end pointing mainly horizontally towards the cutting device (14) during cutting of the hole in the shell, whereupon the apparatus turns the egg (1) to a position with the hole pointing down towards the embryo lifting device (15).

14. An embryonated egg harvesting apparatus according to any one of claims 1 to 13, **characterized in that** the embryo lifting device (15) is tubular and has its end opposite the free end connected to a vacuum chamber (30) for sucking the embryo into the vacuum chamber (30) when the vacuum in the chamber (30) is activated.

15. An embryonated egg harvesting apparatus according to claim 14, **characterized in that** tubular portion (29) of the lifting device (15) has an inner diameter di in the range from 15-20 mm, preferably in the range from 17-19 mm.

16. An embryonated egg harvesting apparatus according to any one of claims 1 to 15, **characterized in that** the apparatus has a plurality of embryo lifting devices (15) for simultaneously treatment of a plurality of eggs (1) in a common operation.

17. An embryonated egg harvesting apparatus according to any one of claims 1 to 16, **characterized in that** the apparatus has a screening station for detecting eggs without an embryo and preferably also a discard device preventing the embryo lifting device (15) from entering such eggs (15).

## Patentansprüche

1. Anlage zur Gewinnung von embryonierten Eiern, die eine Eierhalterung (13) und eine Einsetzungsvorrichtung zur Einführung in das Ei (1) durch ein Loch in der Eierschale aufweist, **dadurch gekennzeichnet, dass** die Einsetzungsvorrichtung eine Embryohebevorrichtung (15) ist, die in das Ei in eine vorgegebene Endposition beweglich ist, in der ein freies Ende der Embryohebevorrichtung innerhalb des Eis (1) in einem Abstand (d) von einem Ende der Eierschale gelagert wird.

2. Anlage zur Gewinnung von embryonierten Eiern nach Anspruch 1, dadurch gekennzeichn e t, dass der Abstand (d) einstellbar ist, vorzugsweise je nach der Größe und/oder dem Alter der Eier (1).

3. Anlage zur Gewinnung von embryonierten Eiern nach Anspruch 1 oder 2, **dadurch gekennzeichnet, dass** die Embryohebevorrichtung (15) durch das Loch in einem unteren Ende der Eierschale nach oben bewegt wird.

4. Anlage zur Gewinnung von embryonierten Eiern nach Anspruch 3, **dadurch gekennzeichnet, dass** der vertikal nach oben vom unteren Ende der Eierschale auf gleicher Ebene wie ein Rand des Lochs gemessene Abstand (d) mindestens 5 mm beträgt.

5. Anlage zur Gewinnung von embryonierten Eiern nach Anspruch 3, **dadurch gekennzeichnet, dass** der vertikal von einem oberen Ende der Eierschale gemessene Abstand (d) mindestens dieselbe Größe beträgt wie die Breite des Embryos.

6. Anlage zur Gewinnung von embryonierten Eiern nach einem der Ansprüche 1 bis 5, **dadurch gekennzeichnet, dass** die Embryohebevorrichtung (15) zum Durchdringen einer Eimembran (3) ausgebildet ist.

7. Anlage zur Gewinnung von embryonierten Eiern nach Anspruch 6, **dadurch gekennzeichnet, dass** das freie Ende der Embryohebevorrichtung (15) spitz ist.

8. Anlage zur Gewinnung von embryonierten Eiern nach einem der Ansprüche 1 bis 7, **dadurch gekennzeichnet, dass** die Embryohebevorrichtung (15) mit mindestens einem Ablaufbereich ausgebildet ist, der sich vom freien Ende nach unten erstreckt.

9. Anlage zur Gewinnung von embryonierten Eiern nach einem der Ansprüche 1 bis 8, **dadurch gekennzeichnet, dass** die Embryohebevorrichtung (15) zur Bewegung in Richtung der Endposition gesteuert wird, um in einer ersten Bewegung die Schalenmembran (3) zu durchstoßen, gefolgt durch ein Herausziehen der Vorrichtung aus dem Loch, und wahlweise gefolgt durch eine anschließende zweite Bewegung bis zur Endposition.

10. Anlage zur Gewinnung von embryonierten Eiern nach einem der Ansprüche 1 bis 9, **dadurch gekennzeichnet, dass** eine Eindringvorrichtung zur Bewegung in Richtung der Endposition gesteuert wird, um die Schalenmembran (3) zu durchstoßen, gefolgt durch ein Herausziehen der Vorrichtung aus dem Loch, und dass die Hebevorrichtung (15) in die Endposition bewegt wird.

11. Anlage zur Gewinnung von embryonierten Eiern nach einem der Ansprüche 1 bis 10, **dadurch gekennzeichnet, dass** die Embryohebevorrichtung (15) für eine vorgegebene Verweilzeit ins Ei (1) eingesetzt wird.

12. Anlage zur Gewinnung von embryonierten Eiern nach einem der Ansprüche 1 bis 11, **dadurch gekennzeichnet, dass** eine Schneidvorrichtung (14) an der Anlage vor Einsetzung der Embryohebevorrichtung (15) in das Ei (1) das Loch in der Eischale schneidet, wobei die Schneidvorrichtung (14) vorzugsweise die Schale öffnet, jedoch die Schalenmembran (3) intakt hinterlässt.

13. Anlage zur Gewinnung von embryonierten Eiern nach Anspruch 12, **dadurch kennzeichnet, dass** das Ei (1) während des Schneidens des Lochs in der Schale mit seinem Ende vorwiegend horizontal zur Schneidvorrichtung (14) weisend gehalten wird, wonach die Anlage das Ei (1) in eine Position umkippt, in der das Loch nach unten zur Embryohebevorrichtung (15) weist.

14. Anlage zur Gewinnung von embryonierten Eiern nach einem der Ansprüche 1 bis 13, **dadurch gekennzeichnet, dass** die Embryohebevorrichtung (15) rohrförmig ist und ihr sich gegenüber dem freien Ende befindliches Ende mit einer Vakuumkammer (30) verbunden ist, um bei Aktivierung des Vakuums in der Kammer (30) den Embryo in die Vakuumkammer (30) zu saugen.

15. Anlage zur Gewinnung von embryonierten Eiern nach Anspruch 14, **dadurch gekennzeichnet, dass** das rohrförmige Abschnitt (29) der Hebevorrichtung (15) einen Innendurchmesser dᵢ im Bereich von 15-20 mm, vorzugsweise im Bereich von 17-19 mm, aufweist.

16. Anlage zur Gewinnung von embryonierten Eiern nach einem der Ansprüche 1 bis 15, **dadurch gekennzeichnet, dass** die Anlage eine Mehrzahl von Embryohebevorrichtungen (15) zur gleichzeitigen Behandlung einer Mehrzahl von Eiern (1) in einem gemeinsamen Vorgang aufweist.

17. Anlage zur Gewinnung von embryonierten Eiern nach einem der Ansprüche 1 bis 16, **dadurch gekennzeichnet, dass** die Anlage eine Screeningstation zum Auffangen von Eiern (1) ohne einen Embryo und vorzugsweise auch eine Ausstoßvorrichtung zur Verhinderung des Eindringens der Embryohebevorrichtung (15) in solche Eier aufweist.

## Revendications

1. Dispositif de récolte d'oeufs embryonnés comprenant un support (13) d'oeuf (1) et un dispositif d'insertion pour entrer dans l'oeuf à travers un trou dans la coquille d'oeuf, **caractérisé en ce que** le dispositif d'insertion est un dispositif de levage d'embryon (15) susceptible de se déplacer dans l'oeuf à une position finale prédéterminée où un bout libre sur le dispositif de levage d'embryon est situé à l'intérieur de l'oeuf (1) à une distance (d) d'un bout de la coquille d'oeuf.

2. Dispositif de récolte d'oeufs embryonnés selon la revendication 1, **caractérisé en ce que** ladite distance (d) est réglable, préférablement réglable dépendant des dimensions et/ou de l'age des oeufs (1).

3. Dispositif de récolte d'oeufs embryonnés selon la revendication 1 ou 2, **caractérisé en ce que** le dispositif de levage d'embryon (15) est déplace vers le haut à travers le trou dans un bout inférieur de la coquille d'oeuf.

4. Dispositif de récolte d'oeufs embryonnés selon la revendication 3, **caractérisé en ce que** ladite distance (d) est au moins 5 mm, mesurée de manière verticale vers le haut depuis le bout inférieur de la coquille d'oeuf au niveau d'un bord du trou.

5. Dispositif de récolte d'oeufs embryonnés selon la revendication 3, **caractérisé en ce que** ladite distance (d) mesurée de manière verticale depuis un bout supérieur de la coquille d'oeuf présente au moins les mêmes dimensions que la largeur de l'embryon.

6. Dispositif de récolte d'oeufs embryonnés selon l'une quelconque des revendications 1 à 5, **caractérisé en ce que** le dispositif de levage d'embryon (15) est conçu pour percer une membrane d' oeuf (3).

7. Dispositif de récolte d'oeufs embryonnés selon la revendication 6, **caractérisé en ce que** le bout libre du dispositif de levage d'embryon (15) est pointu.

8. Dispositif de récolte d'oeufs embryonnés selon l'une quelconque des revendications 1 à 7, **caractérisé en ce que** le dispositif de levage d'embryon (15) est pourvu d'au moins une zone de drainage s'étendant vers le bas depuis le bout libre.

9. Dispositif de récolte d'oeufs embryonnés selon l'une quelconque des revendications 1 à 8, **caractérisé en ce que** le dispositif de levage d'embryon (15) est commandé au déplacement dans la direction de la position finale dans un premier déplacement pour percer la membrane (3) de coquille, suivi par un retrait du dispositif en dehors du trou, et facultativement suivi par un deuxième déplacement subséquent continu à la position finale.

10. Dispositif de récolte d'oeufs embryonnés selon l'une quelconque des revendications 1 à 9, **caractérisé en ce qu'**un dispositif de pénétration est commandé au déplacement dans la direction de la position finale pour percer la membrane (3) de coquille, suivi par un retrait du dispositif en dehors du trou, et **en ce que** le dispositif de levage (15) est déplacé à la position finale.

11. Dispositif de récolte d'oeufs embryonnés selon l'une quelconque des revendications 1 à 10, **caractérisé en ce que** le dispositif de levage d'embryon (15) est inséré dans l'oeuf (1) pour une durée de maintien prédéterminée.

12. Dispositif de récolte d' oeufs embryonnés selon l'une quelconque des revendications 1 à 11, **caractérisé en ce qu'**un dispositif à découper (14) arrangé sur le dispositif de récolte coupe le trou dans la coquille d'oeuf avant l'insertion du dispositif de levage d'embryon (15) dans l'oeuf (1) dont la coquille est préférablement découpée par le dispositif à découper (14), tandis que la membrane (3) de coquille reste intacte.

13. Dispositif de récolte d'oeufs embryonnés selon la revendication 12, **caractérisé en ce que** l'oeuf (1) est retenu avec son bout orienté essentiellement horizontalement vers le dispositif à découper (14) lors du découpage du trou dans la coquille, et ensuite le dispositif tourne l'oeuf (1) à une position avec le trou orienté vers le bas vers le dispositif de levage d'embryon (15).

14. Dispositif de récolte d'oeufs embryonnés selon l'une quelconque des revendications 1 à 13, **caractérisé en ce que** le dispositif de levage d'embryon (15) est tubulaire et présente son bout opposé du bout libre relié à une chambre à vide (30) pour conduire par aspiration l'embryon dans la chambre à vide (30) lorsque le vide dans la chambre (30) est activé.

15. Dispositif de récolte d'oeufs embryonnés selon la revendication 14, **caractérisé en ce que** la portion tubulaire (29) du dispositif de levage (15) présente un diamètre intérieur dᵢ entre 15-20 mm, préférablement entre 17-19 mm.

16. Dispositif de récolte d'oeufs embryonnés selon l'une quelconque des revendications 1 à 15, **caractérisé en ce que** le dispositif présente une multitude de dispositifs de levage d'embryon (15) pour traitement simultané d'une multitude d'oeufs (1) dans un procédé commun.

17. Dispositif de récolte d'oeufs embryonnés selon l'une quelconque des revendications 1 à 16, **caractérisé en ce que** le dispositif présente une station de criblage pour détecter des oeufs (1) sans un embryon et préférablement aussi un dispositif d'écartement empêchant le dispositif de levage d'embryon (15) d'entrer dans de tels oeufs.
